# EUROPEAN PATENT SPECIFICATION

(11) **EP 0 500 727 B1**
(45) Date of publication and mention of the grant of the patent: **21.01.1998**
(21) Application number: 90917320.5
(22) Date of filing: 13.11.1990
(51) Int. Cl.: G01N 33/569, C12Q 1/68, C12Q 1/00, C12N 5/00

(54) **NON-INVASIVE METHOD FOR ISOLATION AND DETECTION OF FETAL DNA**
EIN NICHTINVASIVES VERFAHREN ZUR TRENNUNG UND ZUM NACHWEIS VON FETALER DNA
PROCEDE NON ENVAHISSANT D'ISOLATION ET DE DETECTION D'ADN F TAL

(30) Priority: 13.11.1989 US 436057
(43) Date of publication of application: 02.09.1992
(60) Divisional application: 97200874.2
(73) Proprietor: CHILDREN'S MEDICAL CENTER CORPORATION, Boston Massachusetts 02115 (US)
(72) Inventor: BIANCHI, Diana, W., Brookline, MA 02146 (US)
(74) Representative: Holdcroft, James Gerald, Dr.
(86) International application number: US9006623
(87) International publication number: WO9107660

(56) References cited:
- WO-A-90/06509
- HISTOCHEMICAL JOURNAL, vol. 19, 1987; U.W. MUELLER et al., pp. 288-296/
- THE LANCET, 1984; A.E. COVONE et al., pp. 841-843/
- THE LANCET, vol. 336, 1990; U.W. MUELLER et al., pp. 197-198/
- JOURNAL OF IMMUNOLOGICAL METHODS, vol. 91, 1986; R.J. BERENSON et al., pp. 11-19/
- CHEMICAL ABSTRACTS, vol. 103, 09 December 1985, Columbus, OH (US); B.H. BUTTERWORTH et al., p. 346, no. 192486c/
- Am J Human Genetics 45 (1989) A252
- Pediatric Res 25 (1989) 139A Abs No 818
- Prenatal Diagnosis 8 (1988) 591
- Proc Natl Acad Sci USA 76 (1979) 1453
- Cytometry 8 (1987) 197
- Prenatal Diagnosis 1 (1981) 61
- Lancet ii (1989) 1363

## Description

### Funding

Work described herein was supported by the National Institutes of Health and Children's Hospital Medical Center.

### Background

A variety of fetal cell types--platelets, trophoblasts, erythrocytes and leucocytes--cross the placenta and circulate transiently within maternal blood (Schroder, J., J. Med. Genet., 12:230-242 (1975); Douglas G.W. et al., Am. J. Obstet. Gynec., 78:960-973 (1959)). There have been numerous reports of efforts to separate fetal cells from maternal cells present in maternal blood, but none has been successful in isolating cells subsequently shown to contain fetal DNA. Distinguishing fetal cells from maternal cells has not been successful for several reasons, including the small number of fetal cells in a maternal blood sample and the fact that morphological differences are slight (e.g., trophoblasts are the only fetal cells which can be distinguished from maternal cells by morphology alone).

Others report screening the peripheral blood of pregnant women for cells of fetal origin. Fetal identification relied on the presence of a single cytogenetic marker, the Y chromosome. Lymphocytes with a putative "XY" karyotype were found in the maternal circulation as early as 14 weeks gestation (Walknowska, J., et al., The Lancet, 1119-1122 (1979)).

The availability of flow cytometry has led many to suggest that fetal cells could be obtained through the use of a flow cytometer and that such cells could be exploited for prenatal genetic diagnosis. However, although cells sorted in this manner have been said to be of fetal origin, based on analysis of cell surface antigens, morphology, or cytogenetic criteria, there had not been confirmation that the cells contain fetal DNA. A method by which fetal DNA could be obtained from maternal blood during pregnancy would be valuable, particularly if it made it possible to carry out prenatal diagnosis by a noninvasive technique.

Am J Human Genetics 45 (1989) A252 and Pediatric Res 25 (1989) 139A Abs. No. 818 both disclose enrichment of maternal blood samples in fetal nucleated erythrocytes by flow sorting, followed by crude extraction of fetal DNA prior to amplification by PCR and Southern blotting with a Y-chromosome specific probe.

### Disclosure of the Invention

The present invention is an in vitro method of separating or isolating fetal DNA present in the blood of a pregnant woman from maternal DNA, as well as an in vitro method of detecting the presence of and/or quantitating selected fetal DNA in fetal DNA, which is useful as a noninvasive prenatal diagnostic or analytical method.

In the present method, fetal nucleated erythrocytes may be isolated from a maternal blood sample by means of a detectable material which binds to the fetal nucleated erythrocytes but not to maternal cells and is then separated from the maternal sample, resulting in enrichment of the fetal nucleated erythrocytes in the sample.

In one embodiment of the present method of isolation, at least one detectably labelled monoclonal antibody specific for an antigen present on fetal nucleated erythrocytes but not for an antigen present on maternal cells, is combined with a maternal blood sample and, once bound to fetal nucleated erythrocytes, is separated from the maternal sample. Alternatively, at least one detectably labelled monoclonal antibody specific for an antigen present on maternal cells, but not for an antigen present on fetal nucleated erythrocytes is used. In a further embodiment, the two types of monoclonal antibodies are used.

In the case in which the detectable label is a fluorescent molecule, separation is carried out by means of flow cytometry, in which fluorescently-labelled molecules are separated from unlabelled molecules. This results in separation of fetal nucleated erythrocytes from maternal cells and, thus, of fetal DNA from maternal DNA. That this separation has occurred can be verified using known techniques, such as microscopy or detection of fetal hemoglobin.

In the present method of determining the occurrence of a selected DNA sequence (or sequences), cells isolated as described above are sorted onto a solid support, such as a slide, and screened for chromosomal abnormalities using in situ hybridization. In this embodiment, a selected nucleic acid probe, such as a labelled DNA probe for chromosomal DNA associated with a congenital abnormality, is combined with the fetal DNA, under conditions appropriate for hybridization of complementary sequences to occur. Detection and/or quantitation of the labelled probe after hybridization results in detection and/or quantitation of the fetal DNA to which the probe has hybridized.

The present method of detecting the occurrence of selected fetal DNA is useful for prenatal evaluation or diagnostic purposes, such as determination of the sex of the fetus, assessment of chromosomal abnormalities and determination of the presence of abnormal genes associated with human disease.

A particular advantage of the method of the present invention by which fetal nucleated erythrocytes are isolated is that such cells can be reliably separated from cells of maternal origin. In addition, because such cells are nucleated and, thus, contain a full complement of fetal genes, the present method makes available complete fetal DNA. The present method of detecting and/or quantitating a selected fetal DNA sequence is particularly valuable not only because of the advantages associated with the present method of isolating fetal cells, but also because it is a noninvasive technique which can be applied early in gestation.

### Brief Description of the Drawings

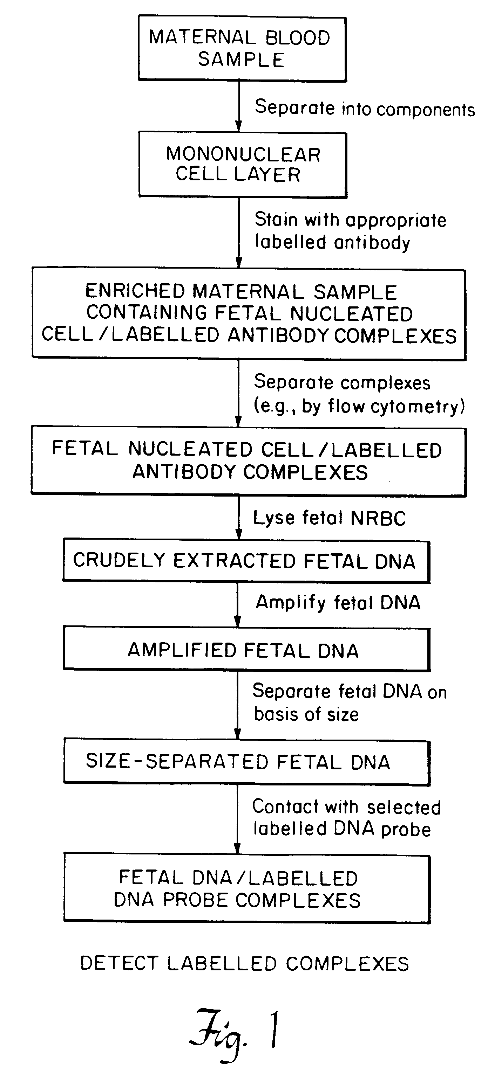

Figure 1 is a schematic representation of the method of the present invention by which fetal nucleated cells are isolated from maternal cells and DNA within the fetal cells is assessed for the occurrence of a particular fetal DNA sequence.

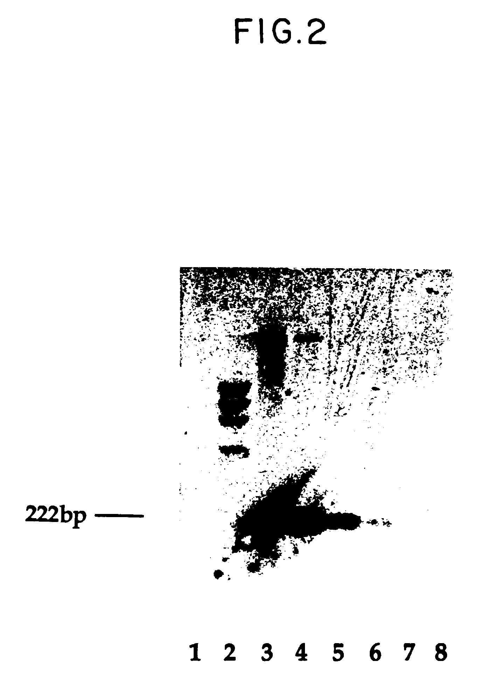

Figure 2 is an autoradiograph of diluted male DNA amplified for 222 bp sequence. Lane 1: reagent control; lane 2: φX174 molecular weight standard; lane 3: 100 ng; lane 4: 10 ng; lane 5: 1 ng; lane 6: 200 pcg.; lane 7: 10 pcg; lane 8: 1 pcg.

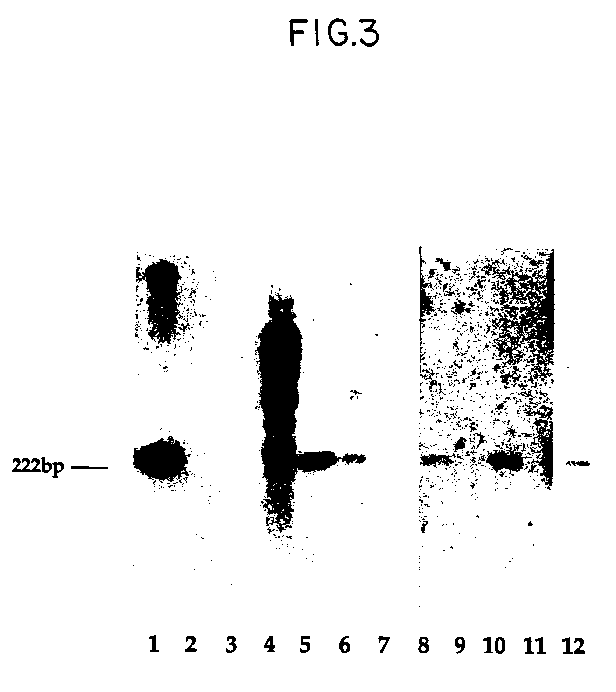

Figure 3 is a composite autoradiograph of amplified patient DNA. Lane 1: 10 ng normal male; lane 2: 10 ng normal female; lane 3: reagent control; lane 4: φX174; land 5: sorted cells from patient 1 (male fetus); lane 6: sorted cells from patient 2 (male fetus); lane 7: sorted cells from patient 3 (female fetus); lane 8: sorted cells from patient 6 (female fetus); lane 9: sorted cells from patient 7 (male fetus); lane 10: sorted cells from patient 8 (male fetus); lane 11: sorted cells from patient 9 (female fetus); lane 12: cord blood from female infant whose cells were prenatally sorted in lane 8.

Figure 4 is a diagram demonstrating the detection of Y chromosomal DNA sequences at various points of gestation in women bearing male pregnancies.

Figure 5 is a series of histograms (A through F) obtained when FITC-anti transferrin receptor was used to determine the presence of mononuclear cells in samples from non-pregnant females to which male cells have been added.

Figure 6 is a composite autoradiograph of amplified male DNA detected in TfR⁺ cells when 10²-10⁶ male cells are added to samples from non-pregnant females and in TfR⁻ cells when 10⁵-10⁶ male cells are added to samples from non-pregnant females.

Figure 7 is a series of histograms (A through H) obtained when anti HPCA-1 antibody was used to determine the presence of mononuclear cells in samples from non-pregnant females to which male cells have been added.

Figure 8 is a photograph illustrating a fluorescent cell due to the positive results of in situ hybridization of the pDP97 probe for the Y chromosome to a fetal nucleated red blood cell.

### Detailed Description of the Invention

The present invention relates to the subject matter of the claims.

The invention provides a noninvasive approach to detect and/or quantitate fetal DNA, such as that associated with a disease or a condition whose assessment during gestation is desired. It also provides a noninvasive means by which the sex of a fetus can be determined.

The following is a description of the basis for the subject method; of the present method of isolating nucleated fetal cells present in the blood of a pregnant woman from maternal cells and, subsequently, separating fetal DNA from maternal DNA; and of the present method of prenatal determination of the occurrence (presence/absence or quantitation) of selected DNA in fetal cells.

### Nucleated erythrocyte as a potential source of fetal genes

It has now been determined that fetal nucleated cells, present in the blood of a pregnant woman are a source of fetal genes. That is, it has been shown that fetal nucleated erythrocytes (also referred to as fetal NRBC) can be isolated or separated from maternal blood and that DNA present in the isolated fetal cells can be used to assess fetal characteristics (e.g., sex, presence or absence of chromosomal abnormalities).

Fetal nucleated erythrocytes were selected for sorting based on the following rationale:
1. In any given fetomaternal hemorrhage, no matter how small, the ratio of fetal erythrocytes to fetal lymphocytes should remain the same as in whole fetal blood; thus, there would be 1,000 times as many red cells as white cells available for analysis.
2. Normal pregnant females do not usually have circulating NRBC; therefore, an isolated NRBC would a priori have a greater chance of being fetal in origin.
3. The majority of pregnancies are blood group compatible, which means that the "transfused" NRBC would probably be tolerated by the mother and remain in her circulation.
4. Because they are nucleated, the NRBC contain a full complement of fetal genes.

### Advances in molecular biology applied to fetal cell sorting

Recent advances in molecular biology have had an enormous impact on the feasibility of fetal cell identification. For example, fluorescent in situ hybridization can be used for this purpose.

The development of the polymerase chain reaction (PCR) (Mullis, K., et al., Cold Spring Harb. Symp. Quant. Biol., 51:263-272 (1986)), with its capacity for DNA analysis from a single cell (Li, H., et al., Nature, 355:414-417 (1988); Handyside, A.H., et al., Lancet 1:347-349 (1989)), has eliminated the technical problems associated with the small number of fetal cells in maternal blood. It makes DNA diagnosis from a single cell possible.

As described below, fetal nucleated erythroblasts have been shown to be present in blood obtained from pregnant women, thus making maternal blood a useful/reliable potential source of fetal DNA; fetal nucleated cells have been distinguished from maternal cells on the basis of surface antigenic characteristics, thus making it possible to separate the two cell types from one another; and fetal DNA present in the separated fetal nucleated cells has been analyzed and characterized.

### Detection of fetal gene sequences in maternal blood

One of the first steps in developing the present method of isolating fetal nucleated cells from the maternal blood supply was identification of monoclonal antibodies that permit identification and separation of fetal cells from maternal cells present in blood obtained from a pregnant woman. This has been done, as described in detail in the Examples. As a result, it has been determined that monoclonal antibodies which recognize maternal leucocytes and monoclonal antibodies which recognize fetal cell surface antigens are useful in separating maternal and fetal cells. The following is a brief description of monoclonal antibodies which have been shown to be useful in separating fetal nucleated cells from maternal cells present in a maternal blood sample. However, other monoclonal antibodies which distinguish between fetal and maternal cells on the basis of surface antigenic differences, can also be used in the present method. The present method requires the use of at least one type of antibody which is specific for (or recognizes) a surface antigen present on fetal nucleated cells, for a surface antigen present on maternal cells, but not specific for both. That is, the present method can be carried out using one or more antibody which distinguishes fetal nucleated cells from maternal cells. The present method can be carried out using whole blood or blood treated or processed to enrich for (increase the concentration of) fetal nucleated cells.

Described below is the selection and successful use of monoclonal antibodies which distinguish fetal nucleated erythrocytes from maternal cells. It is to be understood, however, that in a similar manner, monoclonal antibodies which make it possible to select for another fetal nucleated cell type (or types) can be identified and used in the present method to separate fetal nucleated cells from maternal cells (and, thus, fetal DNA sources from maternal DNA).

Initial efforts focused on the elimination of contaminating maternal leucocytes in the mononuclear cell layer and identification of monoclonal antibodies effective in carrying out this separation, which results in production of a maternal sample enriched in fetal nucleated cells.

HLe-1 (Becton-Dickinson Monoclonal center, Mountain View, CA, catalog #7463) is a monoclonal antibody available as a direct fluorescein isothiocyanate (FITC) conjugate. It recognizes an antigen present on mature human leucocytes and on very immature erythrocyte precursors, but not on mature nucleated erythrocytes (Loken, M.E., et al., Blood, 69:255-263 (1987)). Thus, maternal leucocytes are recognized and bound, but fetal nucleated erythrocytes are not, making separation of the two possible. As described in detail in Example 1, this labelled antibody was used to eliminate maternal leucocytes in the mononuclear cell layer.

As is also described (Example 1), a combination of monoclonal antibodies has been used for the same purpose (i.e., elimination of maternal cells from the blood sample). As described, anti-monocyte antibody (M3) and anti-lymphocytes antibody (L4) have been used to remove maternal cells from the mononuclear cell layer resulting from density gradient centrifugation.

Monoclonal antibodies which recognize fetal nucleated cells but do not recognize maternal cells were also identified. As described in detail in Example 1, a monoclonal antibody which recognizes the transferrin receptor was identified. Erythroblasts have been shown to express the transferrin receptor (Loken, M.R., et al., Blood, 69:255-263 (1987)) antigen on their cell surfaces from the BFU-E stage until nuclear extrusion (Loken, M.R. et al., Blood, 69:255-263 (1987)). The transferrin receptor is also present on activated lymphocytes (Trowbridge, I.S. and M.B. Omary, Proc. Natl. Acad. Sci. USA, 78:3039-3043 (1981)), certain tumor cells (Greaves, M. et al., Int. J. Immunopharmac., 3:283-300 (1981)), and trophoblast cells (Galbraith, G.M.P. et al., Blood, 55:240-242 (1980)). Thus, such an antibody is specific for or recognizes (binds to) fetal nucleated cells, but not maternal leucocytes. As described in Example 1, commercially available fluorescein-conjugated monoclonal antibodies against the transferrin receptor (TfR) were used to separate fetal nucleated erythrocytes from maternal cells. Although the antibody is not specific for fetal nucleated erythrocytes, it facilitated their enrichment in the flow-sorted samples. Other monoclonal antibodies which are able to distinguish between fetal nucleated cells and maternal cells present in a blood sample can also be used. Such antibodies include commercially available monoclonal antibodies and those which can be produced using known techniques.

Separation of fetal nucleated cells from a maternal blood sample using antibodies described above can be carried out with samples of whole blood or a fraction of whole blood (i.e., one resulting from treatment or processing of whole blood to increase the proportion of fetal nucleated cells present, referred to as an enriched maternal sample. An enriched maternal sample is produced, for example, in a two-step process. The maternal sample is subjected to initial separation on the basis of size, such as by Ficoll-Hypaque density gradient centrifugation. This results in production of a supernatant layer, which contains platelets; a mononucluear cell layer; and an agglutinated pellet which contains non-nucleated erythrocytes and granulocytes. The mononuclear layer is separated from the other layers, to produce a maternal sample which is enriched in fetal nucleated cells.

The maternal sample, whether maternal whole blood or an enriched maternal sample, is subjected to separation, based on surface antigenic differences between fetal nucleated cells and maternal cells using antibodies described above. The maternal sample is contacted with at least one monoclonal antibody which is specific for either fetal nucleated cells or maternal cells, but not for both and, thus, makes it possible to separate the two types of cells. The maternal sample can be combined with a set of two or more monoclonal antibodies, each of which is specific for either fetal or maternal cells, but not for both. The combination of monoclonal antibodies can be designed to enhance separation of the two types of cells (e.g., the combination of anti-TfR antibody and HLe-1 antibody described previously) beyond that possible with a single monoclonal antibody. Separation of the fetal cells is carried out using known techniques, such as flow cytometry, use of immunomagnetic beads and cell panning. In general, the monoclonal antibodies have a detectable label (e.g., radioactive material, fluorophore).

Male (Y-specific) DNA has been detected in cells sorted from pregnant women at various points in gestation. Briefly, the mononuclear cell layer was isolated from venous blood samples obtained from women between 11 and 16 weeks gestation. Separation was carried out using Ficoll/Hypaque density centrifugation, followed by incubation with monoclonal antibodes (Anti-TfR, anti-Leu 4 and anti-Leu^{M}3) conjugated with a fluorescent marker or compound (fluorescein, phycoerythrin) and dual color analysis and flow sorting on a fluorescence-activated cell sorter. The cells that displayed green fluorescence, but not red fluorescence (TfR positive, Leu 4 negative, Leu M3 negative), were fetal nucleated cells and were separated from the remainder of the sample. These cells were lysed, after which the DNA was amplified and probed for the presence of a 397 bp sequence of the Y chromosome.

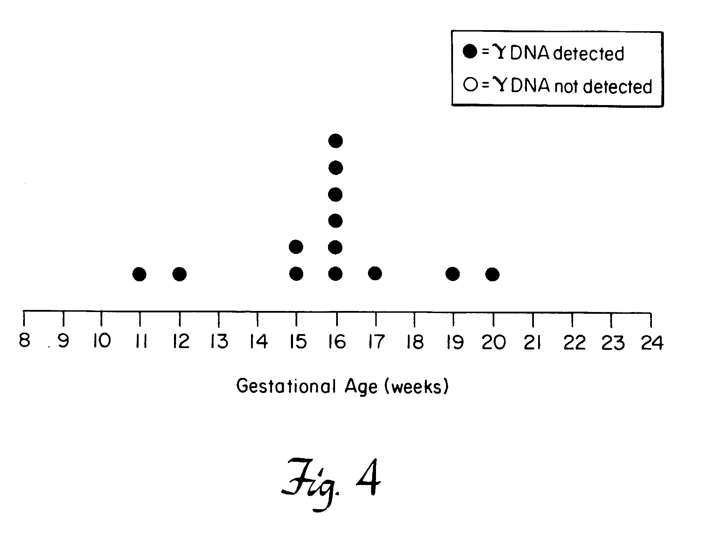

The results presented in Example 6 indicate the procedure allows the detection of the 397 bp sequence present in as little as 5 pg of male DNA. In addition, they suggest that there is a relationship between gestational age and detection of male DNA, as illustrated in Figure 4. This data suggests there may be a biologic "window" for transfer of fetal nucleated erithrocytes into maternal circulation.

DNA sequences from the father can be identified in the autosomal chromosomes of the fetus. Consequently, the method of the present invention can be used to separate female fetal nucleated cells, as well as male fetal nucleated cells, from maternal blood. Thus, the method can be used for all DNA-based diagnostic procedures currently being used in other methods, such as amniocentisis.

Further support for of the present method's capability to identify Y-specific DNA in nucleated erthyroctyes obtained from peripheral blood of pregnant women is given by reconstruction experiments. As described in Example 8, male cord blood was added to blood obtained from non-pregnent females to simulate the presence of fetal cells in maternal blood. Briefly, venous blood samples were collected from healthy, non-pregnant women and the mononuclear cell layers isolated by Ficoll/Hypaque density centrifugation. Mononuclear cells from the umbilical cords of male infants (ranging from 10² to 10⁶ cells) were added to the mononuclear cell layers of the blood of non-pregnant women. The cord blood contains a large percentage of nucleated erythrocytes. The results obtained from these experiments were substantially similar to those obtained from pregnant women at various stages in gestation. Amplified sequences from the Y chromosome, consistent with the presence of male DNA, were detected when 10² male cells were added to the female cells.

### Uses of the Present Method of Fetal Nucleated Cell Isolation and Fetal DNA Characterization

Thus, it has been demonstrated that fetal DNA can be obtained from fetal nucleated cells present in a maternal blood sample. The method is useful as a tool for prenatal assessment (e.g., as a means for assessing chromosomal abnormalities, for determining whether DNA associated with a disease is present, or for detecting Y-specific DNA). It is particularly useful because it is noninvasive and requires only a small sample of blood.

Fetal DNA sequences in fetal nucleated erythrocytes, isolated as described herein or by other means by which fetal nucleated cells can be separated from a maternal blood sample, can be analyzed or assessed for the occurrence of a DNA sequence or DNA sequences (gene(s) or gene portion(s)) which are of interest for diagnostic or other purposes. The DNA sequence(s) or gene(s)/gene portion(s) present in fetal cells are referred to herein as fetal DNA of interest. For example, the selected DNA whose presence or absence is to be determined and whose quantity can also be determined is the gene for a disease, such as cystic fibrosis, where the causative gene or gene portion has been cloned and sequenced; alternatively, it is a probe for X- or Y- specific DNA. The same procedure can also be used, with appropriate modifications (e.g., an appropriate DNA probe, time, temperature), to detect other genes or gene portions.

As used in a diagnostic context, such as to detect the gene known to cause cystic fibrosis, the present method is carried out as follows: Initially, a maternal blood sample (typically 20 ml.) is obtained and separated into component layers based on relative weights (e.g., by Ficoll-Hypaque density gradient centrifugation) to remove non-nucleated erythrocytes and produce a mononuclear cell layer. This results in production of a maternal blood sample enriched in fetal nucleated erythrocytes. The mononuclear cell layer is stained with at least one appropriate monoclonal antibody (e.g., one which is specific for the type of fetal nucleated cell to be separated from the sample). For example, a monoclonal antibody specific for fetal nucleated cells, such as anti-TfR antibody, described above, can be used. In general, the monoclonal antibody used bears a detectable label. Alternatively, a combination of selected labelled monoclonal antibodies, such as monoclonal antibodies specific for fetal nucleated cells (e.g., anti-TfR antibody) and monoclonal antibodies specific for maternal leucocytes (HLe-1 or L4 and M3), each labelled with a different fluorescent compound, can be used to remove essentially all maternal cells. Labelled cells are subsequently separated from one another using a known method, such as flow cytometry. Binding of the monoclonal antibodies to cells for which they are specific results in production of labelled monoclonal antibody-cell complexes. For example, in the case in which anti-TfR antibodies and HLe-1 are used, fetal nucleated erythrocytes are bound by anti-TfR antibody, to produce fetal nucleated erythrocytes/anti-TfR antibody complexes, and maternal leucocytes are bound by HLe-1 antibodies, to produce maternal leucocyte/HLe-1 antibody complexes. The fetal nucleated erythrocyte/anti-TfR antibody complexes are separated from maternal cell/HLe-1 antibody complexes, using, for example, flow cytometry.

The occurrence of fetal DNA associated with diseases or conditions other than cystic fibrosis can also be detected and/or quantitated by the present method. In each case, an appropriate probe is used to detect the sequence of interest. For example, sequences from probes St14 (Oberle, I., et al., New Engl. J. Med., 312:682-686 (1985)), 49a (Guerin, P., et al., Nucleic Acids Res., 16:7759 (1988)), KM-19 (Gasparini, P., et al., Prenat. Diagnosis, 9:349-355 (1989)), or the deletion-prone exons for the Duchenne muscular dystrophy (DMD) gene (Chamberlain, J.S., et al., Nucleic Acids Res., 16:11141-11156 (1988)) are used as probes. St14 is a highly polymorphic sequence isolated from the long arm of the X chromosome that has potential usefulness in distinguishing female DNA from maternal DNA. It maps near the gene for Factor VIII:C and, thus, may also be utilized for prenatal diagnosis of Hemophilia A. Primers corresponding to sequences flanking the six most commonly deleted exons in the DMD gene, which have been successfully used to diagnose DMD by PCR, can also be used (Chamberlain, J.S., et al., Nucleic Acids Res., 16:11141-11156 (1988)). Other conditions which can be diagnosed by the present method include β-thalassemia (Cai, S-P., et al., Blood, 73:372-374 (1989); Cai, S-P., et al., Am. J. Hum. Genet., 45:112-114 (1989); Saiki, R.K., et al., New Engl. J. Med., 319:537-541 (1988)), sickle cell anemia (Saiki, R.K., et al., New Engl. J. Med., 319:537-541 (1988)), phenylketonuria (DiLella, A.G., et al., Lancet, 1:497-499 (1988)) and Gaucher disease (Theophilus, B., et al., Am. J. Hum. Genet., 45:212-215 (1989)). An appropriate probe (or probes) is available for use in the present method for assessing each condition.

It is also possible to separate fetal cells from maternal cells by means other than flow cytometry, as mentioned previously, and to analyze fetal nucleated erythrocyte DNA obtained in this way. Such separation procedures may be used in conjunction with or independent of flow cytometry. This is advantageous because lack of access to a flow cytometer, as well as expense, could limit potential applications of this technique. Thus, other methods of fetal cell separation can be used. The separation method used can result in elimination of unwanted cells ("negative selection") or isolation of rare but desirable cells ("positive selection").

For example, separation by immunomagnetic beads or by cell panning can be used. In this embodiment, the mononuclear cell layer is isolated, as described previously. This layer is then mixed with antibody-coated polymer particles containing magnetic cores (e.g., "Dynabeads"). These immunomagnetic beads are available coated with a variety of antibodies. For example, immunomagnetic beads coated with antibody to leucocyte antigens and antibody to mouse immunoglobulins, which can be subsequently conjugated to mouse monoclonal antibody against the human transferrin receptor, can be used. After mixing, the rosetted cells are isolated with a magnetic particle concentrator. In one embodiment, two sets of antibody-coated immunomagnetic beads are used in succession. First, the maternal leucocytes are depleted and then the remaining TfR positive cells are collected. Subsequent steps in the method (amplification, separation, contact with an appropriate DNA probe or probe set) are as described for cells separated by flow cytometry.

Mueller et al. (Lancet, 336: 197-200 (1990)) have described a method of isolating placenta-derived trophoblast cells in the blood of pregnant women using magnetic beads. This method included mixing 1 ml of monoclonal antibody hybridoma culture supernatant with 2 x 10⁷ magnetic beads precoated with sheep antibody to mouse IgG (Fc fragment) (Dynabeads M-450, Dynal AS, Oslo, Norway) and incubated overnight at room temperature. The coated beads were stored at 4°C and washed three times in ice-cold RPMI 1640 medium containing lithium heparin (10 IU/ml). The blood from the pregnant women was collected into tubes containing 10 IU of lithium per ml of whole blood, diluted 1:10 with RPMI containing lithium, and incubated with the antibody coated beads at 4°C overnight. The desired cells were bound to the antibody on the bead; the beads collected by means of a cobalt-samarium magnet. Although in this case the antibody was directed against trophoblast antigens, a similar technique can be utilized with, for example, antibody to cell surface antigens present on fetal nucleated erythrocytes and not present on maternal cells. An advantage to this particular technique is that an initial step which results in mononuclear cell isolation is not added. Additionally, the magnetic beads can be used for both positive (fetal cells) and negative (maternal cells) selection.

An alternative method of isolation can be a modification of the method described by R.J. Berenson et al. (J. of Immunol. Methods, 91: 11-19 (1986)) in which the high affinity between the protein avidin and the vitamin biotin was exploited to create an indirect immunoadsorptive procedure. In this technique, avidin was linked to cyanogen bromide activated sepharose 6MB beads and washed in an alternating fashion with coupling buffer (0.1 M NaHCO₃ in 0.5 M NaCl at pH 8.3) and washing buffer (0.1 M sodium acetate in 0.5 M NaCl at pH 4.5) and stored at 4°C. The blood cells were incubated with 1) murine monoclonal antibody, and 2) biotinylated goat anti-mouse immunoglobulin. A 3 ml column of gel was packed in a Pharmacia K 19/15 column. The treated cells were passed through the column in phosphate buffered saline containing 2% bovine serum albumin. Adherent cells were dislodged by mechanical agitation. This technique can be applied to fetal cell separation if the antibodies used recognize fetal cell surface antigens or maternal cell surface antigens, but not both. Variations in methods for conjugating antibodies to beads exist; examples include those described by Thomas and co-workers (Thomas, T.E., et al. (J. of Immuno. Methods, 120: 221-131 (1989)) and by deKretser and co-workers (deKretser, T.A., et al. (Tissue Antigens, 16: 317-325 (1980)). The use of an antibody-bound column does not require the preliminary isolation of the mononuclear cell fraction from whole blood.

Once the fetal cells are isolated from maternal blood, they may be cultured to increase the numbers of cells available for diagnosis, if desired. E. Fibach et al. (Blood, 73: 100-103 (1989)) have described a method that supports the growth of human hematopoietic progenitor cells. This step-wise method involves 1) initial culture in the presence of conditioned medium from human bladder carcinoma cells, 2) removal of leucocytes by harvest of non-adherent cells and lysis with monoclonal antibodies, and 3) reculture of cells in medium supplemented by recombinant erythropoietin.

Other methods of separating fetal nucleated cells from maternal cells can also be used, provided that they make it possible to differentiate between fetal cells and maternal cells, and to isolate one from the other.

A kit for use in carrying out the present method of isolating and detecting fetal DNA of interest, such as a chromosomal abnormality associated with a disease or other condition, in a maternal blood sample can be produced. It includes, for example, a container for holding the reagents needed; the reagents and, optionally, a solid support for use in separating fetal nucleated cell/specific antibody complexes from other sample components or for removing maternal cells complexed with specific antibody. The kit, as indicated, can also include a solid support to be used in separating complexes formed from other samples components. Such solid support can be, for example, a glass slide, nitrocellulose filter, or immunomagnetic beads and can have affixed thereto an antibody selective for the antibody present in the fetal nucleated cell/specific antibody complexes.

The present invention will now be illustrated by the following examples, which are not intended to be limiting in any way.

### EXAMPLE 1 Antibody selection for isolation and sorting of fetal nucleated erythrocytes (NRBCs)

### Removal of maternal leucocytes from maternal blood using human leucocyte antigen (HLe-1)

The technique of fetal NRBC isolation began with an initial Ficoll-Hypaque density gradient centrifugation to remove the tremendously high number of non-nucleated erythrocytes in maternal blood. Peripheral blood was centrifuged and separated into a supernatant layer containing platelets, a mononuclear cell layer, and an agglutinated pellet consisting of non-nucleated erythrocytes and granulocytes. The mononuclear cell layer consisted of lymphocytes, monocytes, possible trophoblasts, and, due to their increased size and density, NRBCs and some reticulocytes. While the Ficoll-Hypaque centrifugation represented an initial enrichment in the proportion of fetal NRBCs present in the maternal sample, flow cytometry and cell sorting was used to improve the purity of the isolated cell population.

The mononuclear cell layer from peripheral blood samples in 63 pregnant women, 15 nonpregnant adults, and 39 umbilical cords, was stained with FITC-HLe-1 for flow cytometric analysis. Umbilical cord samples were used as a substitute for whole fetal blood. Representative histograms displaying fluorescence versus low-angle light scatter (an approximation of cell size) for each of the three groups were generated. Histogram peaks were identified that corresponded to leucocytes, erythrocytes and platelets. In 9 pregnant women, 7 nonpregnant adults and 12 umbilical cord samples, fluorescent (HLe-1 positive) and non-fluorescent (HLe-1 negative) cell populations were sorted for detailed microscopy after Wright-Giemsa staining. While the HLe-1 positive populations were always composed of leucocytes independent of the sample source, the HLe-1 negative populations differed.

In cord blood, the HLe-1 negative cells were non-nucleated and nucleated erythrocytes with occasional platelets. In the pregnant women, there were platelets, non-nucleated erythrocytes, and a very rare NRBC. In non-pregnant adults, only platelets and debris were seen. Thus, cord blood, with its high percentage of NRBCs, was used as a reference to establish cell sorting parameters. Microscopy confirmed the specificity of the antibody-antigen binding and that the sorted HLe-1 negative cells were relatively free from leucocyte contamination. These sorting parameters were utilized to isolate potential fetal NRBC on 40 pregnancies.

### Enrichment of fetal NRBC in maternal blood using transferrin receptor antigen (TfR)

The transferrin receptor (Newman, R., et al., Trends Biochem. Sci. 1:397-399 (1982)) is a surface glycoprotein important in cellular iron transport. The TfR is present on activated lymphocytes (Trowbridge, I.S., et al., Proc. Natl. Acad. Sci. USA, 78:3039-3043 (1981)), certain tumor cells (Greaves, M., et al., Int. J. Immunopharmac., 3:283-300 (1981)), and trophoblast cells (Galbraith, G.M.P., et al., Blood, 55:240-242 (1980)). Erythroblasts express the TfR on their cell surfaces from the BFU-E stage until nuclear extrusion (Loken, M.R., et al., Blood, 69:255-263 (1987)). Thus, TfR is an excellent "candidate antigen" for enrichment of fetal NRBCs found in maternal blood. Monoclonal antibody against TfR is available as both a fluorescein conjugate (Becton-Dickinson catalog #7513) and a phycoerythrin (PR) conjugate (gift of Dr. Michael Loken, Becton-Dickinson). The mononuclear cell layer was isolated from peripheral blood samples in 6 pregnant women, 4 non-pregnant adults, and 3 newborn umbilical cords for TfR analysis and microscopy. Representative histograms of fluorescence versus light scatter from these three groups were generated.

Whereas umbilical cord samples had a large population of fluorescent (TfR positive cells) that were heterogenous in size, non-pregnant adults and pregnant adults had smaller percentages of fluorescent cells that clustered in discrete groups. In addition, there were slight differences in the percentages of TfR positive cells in the pregnant (mean = 0.83) versus non-pregnant (mean = 0.32) samples studies.

Microscope studies of the TfR positive cells were performed using Wright-Giemsa stain for morphology and Kleihauer-Betke technique for the detection of fetal hemoglobin (Kleihauer, E., et al., Klin Wochenschr., 35:637-638 (1957)). In the umbilical cord samples, large numbers of nucleated and non-nucleated erythrocytes containing fetal hemoglobin and occasional leucocytes were identified visually. In the pregnant women, the predominant cell types were nucleated and non-nucleated erythrocytes containing fetal hemoglobin, although leucocytes were infrequently observed. In contrast, the samples from the non-pregnant controls consisted almost exclusively of lymphocytes and monocytes. Because trophoblast cells express TfR, it was postulated that they might be present in the sorted population from the pregnant women; none was detected.

### Dual antibody analysis

Because both antibodies enriched the proportion of NRBCs present, but did not completely exclude other cell types in the sorted samples, combinations of antibodies were used to isolate pure populations of fetal NRBCs. Preliminary dual antibody studies were performed using PE-conjugated TfR and FITC-conjugated HLe-1. NRBCs are TfR positive and HLe-1 negative, whereas maternal leucocytes are HLe-1 positive. These experiments worked well and resulted in separation of maternal leucocytes.

Thus, the work described above defined flow cytometric parameters for enrichment and sorting of NRBCs in peripheral blood from pregnant women. In addition, microscopic studies revealed that morphologic differences occur in mononuclear cell populations derived from venous blood samples in pregnant versus non-pregnant adults.

### EXAMPLE 2 DNA hybridization studies in HLe-1 negative cells sorted from maternal blood

To confirm fetal origin of the cells sorted as described in Example 1, Y chromosomal probes were used because it is the Y chromosome that is unquestionably fetal in origin. The assessments were designed to study whether the presence of Y chromosomal DNA in maternal blood as detected on autoradiographs performed antenatally correlated with the subsequent birth of a male infant.

### DNA isolation

HLe-1 negative cells from cord blood and pregnant women were sorted into test tubes. Conventional methods of DNA isolation as well as modification of cruder methods (Lau, Y-F., et al. Lancet, 1:14-16 (1984); McCabe, E.R.B., et al., Hum Genet., 75:213-216 (1987)) were attempted without success in detecting Y chromosome derived bands on Southern Blots. All were limited by the small numbers of cells present.

### EXAMPLE 3 Direct hybridization to cells deposited on filters

In order to circumvent technical problems associated with DNA isolation, a method of direct DNA hybridization to cells flow sorted onto nitrocellulose filters was developed (Bianchi, D.W., et al., Cytometry, 8:197-202 (1987)). In control experiments, the sex of a newborn was determined from as few as 50 sorted cord blood leucocytes or 5,000 HLe-1 negative cells (a mixture of nucleated and non-nucleated cells).

The methodology was then applied to detection of Y chromosomal sequences in HLe-1 negative cells sorted from peripheral blood samples in 40 women between 8½ and 38 weeks gestation. Results were the following:

| Dot Blot Hybridization with Y Chromosomal Probe | Delivered Male Infant | Delivered Female Infant | Lost to Follow-up |
|---|---|---|---|
| + | 3 | 2 | 0 |
| - | 21 | 12 | 2 |

It was concluded that hybridization with this probe was not predictive of male pregnancy. The possibility exists that there was fetal DNA present on the filters where DNA hybridization occurred, but that this DNA bound to the Y probe nonspecifically. Thus, the filters interpreted as "positive" for male DNA might actually have been "positive" for fetomaternal hemorrhage.

### EXAMPLE 4 Determination of the Volume, Morphology and Universality of Fetomaternal Hemorrhage

### a. General Strategy

It is also possible, because of the availability of the present method of isolating fetal nucleated cells from blood obtained from a pregnant woman, to determine whether fetal cells can be found in the maternal blood in all pregnancies. A data base can be created that can provide information on the number and type of fetal cells circulating in maternal blood as pregnancy progresses. Based on previous work, it is anticipated that there will be a normal range of values that is dependent on gestational age; deviation from these values will be studied as a potential indication of a pregnancy at risk. Specifically, large amounts of fetal blood in the maternal circulation may be correlated with placental abnormalities, threatened miscarriage and intrauterine growth retardation.

Maternal venous blood samples are collected from pregnant women, generally prior to any invasive procedures. In general, a single 20 ml. venous blood sample will be obtained. In a subgroup of patients, permission will be sought to draw blood samples every 4 weeks to follow changes in numbers of fetal cells present. Blood is collected in EDTA, diluted 1:1 with Hanks Balanced Salt Solution (HBSS), layered over a Ficoll-Hypaque column (Pharmacia) and spun at 1400 rpm for 40 minutes at room temperature. The mononuclear cell layer will be isolated, washed twice with HBSS, and stained with fluorescent monoclonal antibodies. For example, this can be a combination of fluorescein isothiocyanate-conjugated antitransferrin receptor (TfR) and phycoerythrin-conjugated anti-monocyte antibodies (M3, Becton-Dickinson catalog #7497) and anti-lymphocyte antibodies (L4, Becton-Dickinson catalog #7347). The staining occurs on ice, in phosphate buffered saline (PBS) containing 2% fetal calf serum and 0.1% sodium azide. The cells are washed in PBS prior to flow cytometry. Analysis and sorting are performed on a Becton-Dickinson FACS-IV interfaced with a Consort 40 program. Data will be acquired on the relative size and fluorescence (in two colors) of the analyzed cells. Cells that are fluorescent in the green wavelength (TfR positive) and not fluorescent in the red wavelength (L4 and M3 negative) will contain the presumed fetal NRBCs. The percentage of these cells in the mononuclear cell layer arerecorded and analyzed as a function of gestational age. These cells are sorted for microscopy and PCR amplification. In addition, cells that are not fluorescent in the green wavelength (TfR negative) but are fluorescent in the red wavelength (L4 and/or M3 positive) are sorted as a presumed maternal leucocyte population and source of maternal DNA polymorphisms.

An additional benefit of studying nucleated fetal cells in maternal blood is that the amount of fetal DNA present can be extrapolated to determine the extent of fetomaternal hemorrhage in normal and unusual pregnancies. In the pregnancies studied, an average amount of 1 ng of fetal DNA (corresponding to 150 NBCRs) was present. Using published values of the number of NRBCs per liter of fetal blood at 16 weeks (3.6 x 10⁹) (Millar, D.S., et al., Prenat. Diagnosis, 5:367-373 (1985); (Forestier, F., et al., Pediatr. Res., 20:342-346 (1986)) and doing simple algebra, these results were calculated to be consistent with 2-20 µl hemorrhage of fetal blood into maternal circulation. This is a trivial amount when compared with the fetoplacental blood volume at 16 weeks, about 20 ml. It is important to validate and extend these results to generate normative data regarding fetomaternal transfusion in early pregnancies. It will be equally important to correlate deviations from the expected results with pregnancy complications.

### Example 5 Reconstruction Experiments Using Non-Pregnant Female Blood and Added Male Cord Blood to Simulate the Presence of Fetal Cells in Maternal Blood

Venous blood samples (20 ml) were collected in EDTA from healthy non-pregnant women. Umbilical cord blood samples (10 ml) were collected in EDTA from normal newborns. The mononuclear cell layer was isolated by Ficoll/Hypaque density centrifugation. Cell counts were performed with a hemocytometer. Separate aliquots of cells were made containing: 1) female cells alone; 2) female cells plus 10² added male cord blood cells; 3) female cells plus 10³ added male cord blood cells; 4) female cells plus 10⁴ added male cord blood cells; 5) female cells plus 10⁵ added male cord blood cells; 6) female cells plus 10⁶ added male cord blood cells; 7) male cord blood cells alone. The separate aliquots were then incubated with the individual monoclonal antibodies being tested. Analysis and sorting were performed using a flow cytometer. For each aliquot, a bivariate histogram was obtained, and gating parameters were established for antibody positive and antibody negative cells. The sorted cells were collected into sterile micro test tubes and frozen at -20°C. PCR amplification was performed with primers that detect a 397 bp sequence unique to the Y chromosome. The presence of a band at 397 bp in autoradiographs was used to confirm the presence of male unbilical cord blood cells in sorted samples.

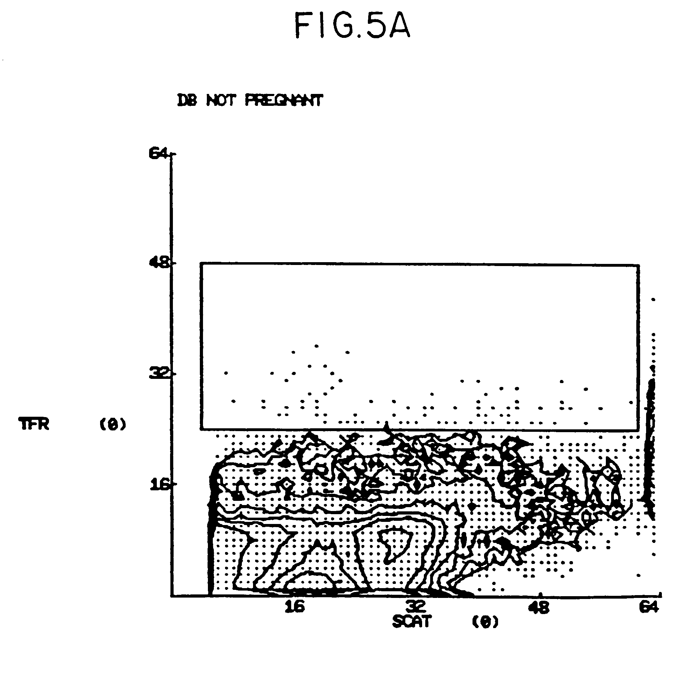

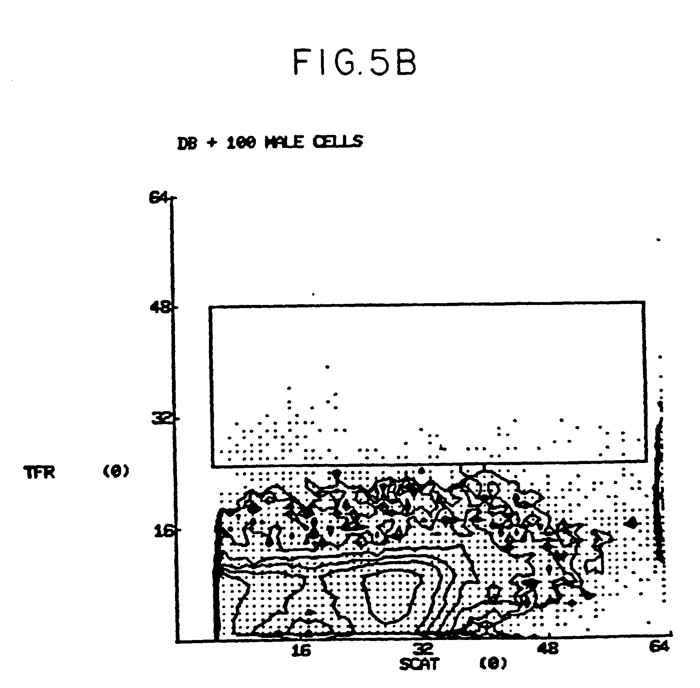

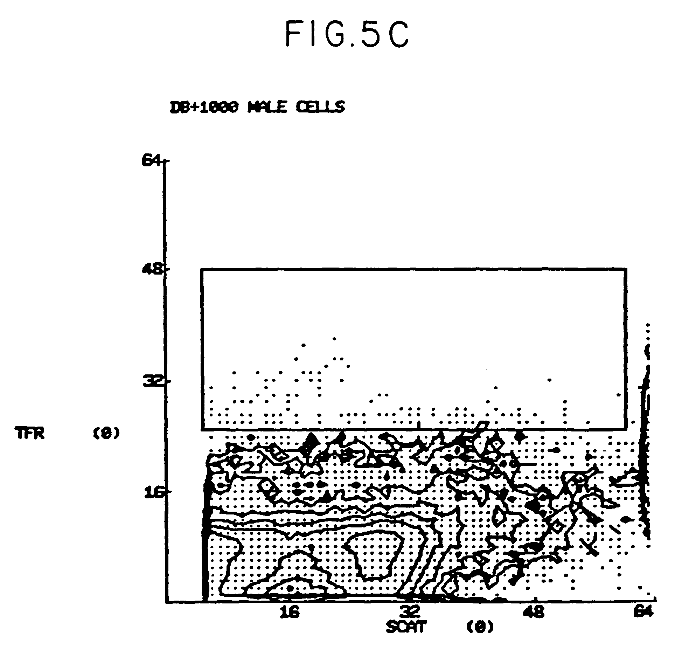

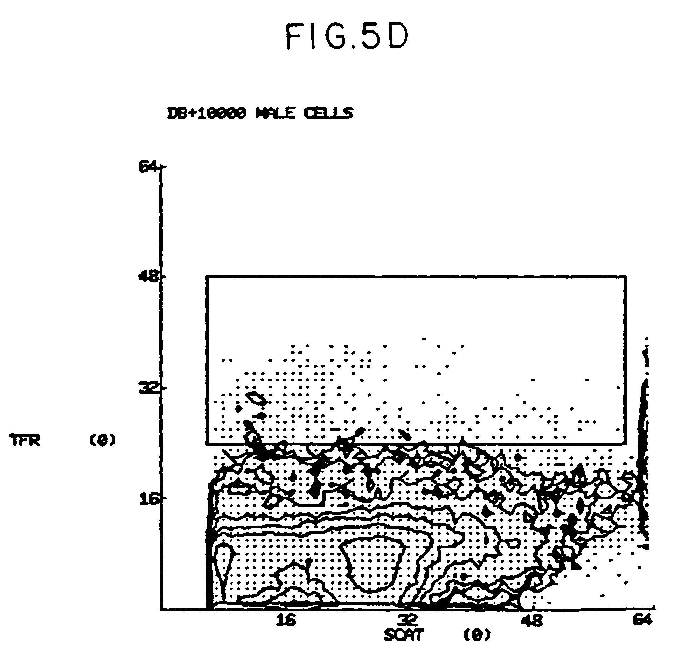

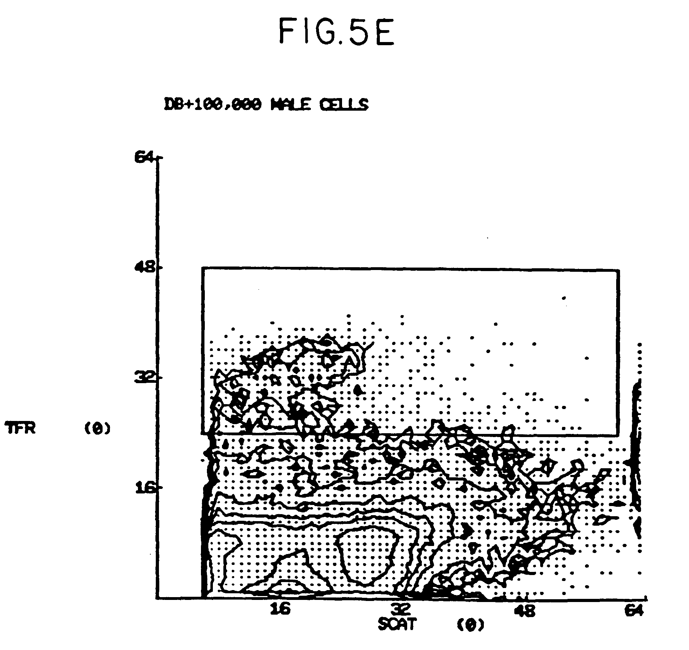

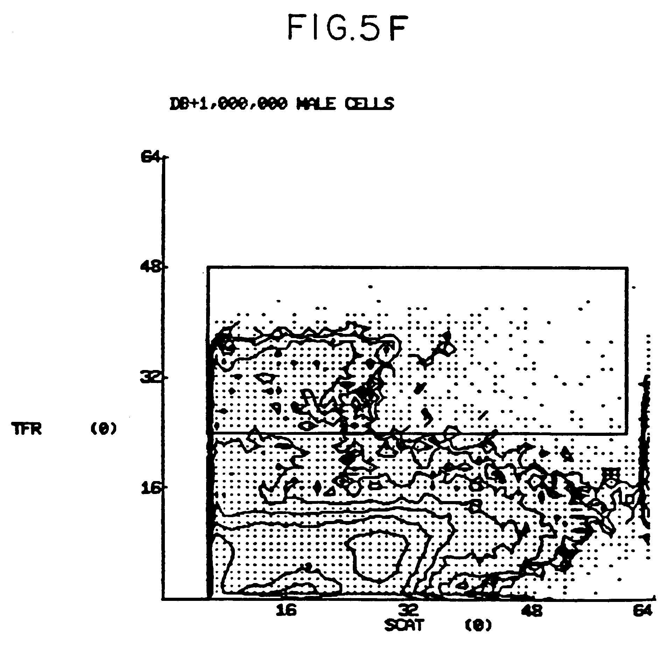

Figure 5 shows the histograms obtained when FITC-anti transferrin receptor is used. In the non-pregnant female, 0.1% of the mononuclear cells react with the antibody. In male cord blood, 24.9% of the mononuclear cells react with the antibody. With the addition of more and more umbilical cord cells to the non-pregnant female cells, an increased percentage of cells that react with the antibody is seen.

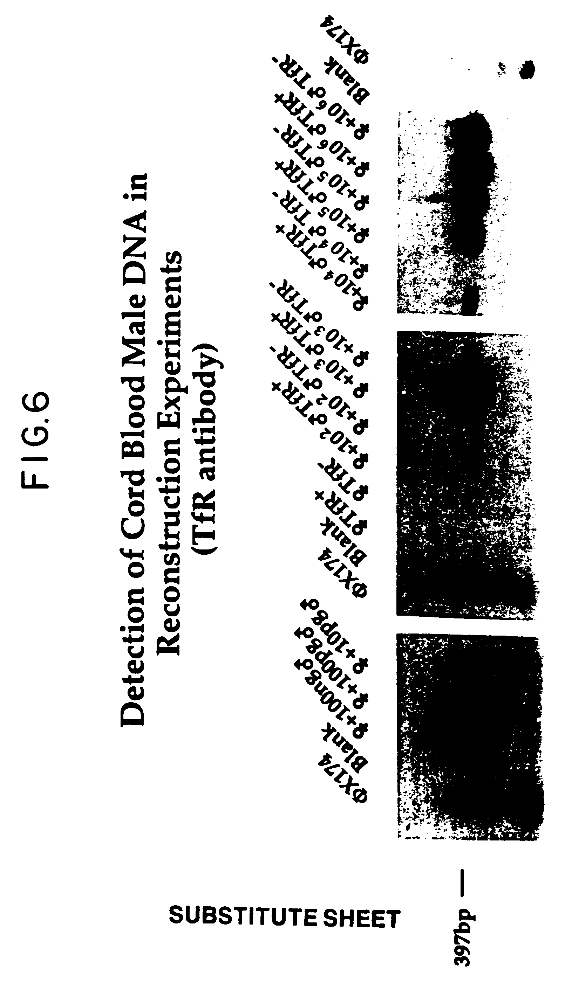

Figure 6 shows that male DNA is detected in the TfR⁺ cells when 10²-10⁶ male cells are added. Male DNA is detected in the TfR⁻ cells when 10⁵-10⁶ male cells are added. This results from the presence of male white blood cells in the TfR⁻ population.

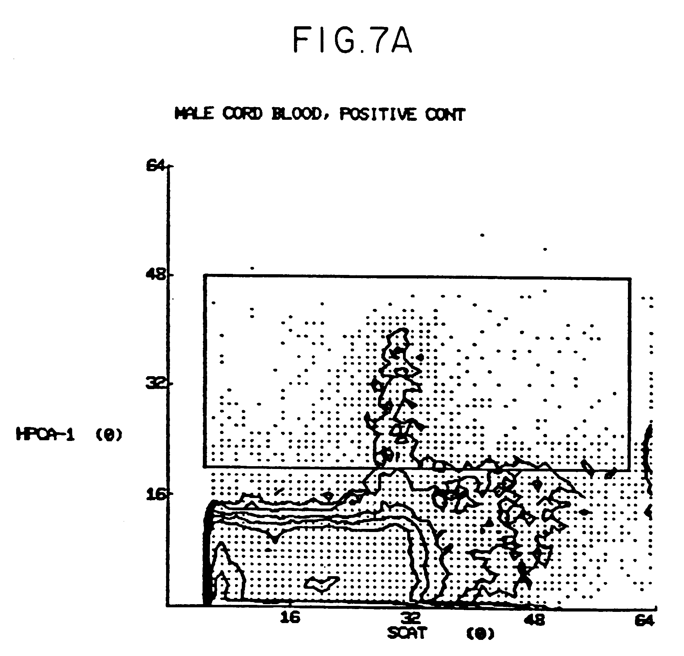

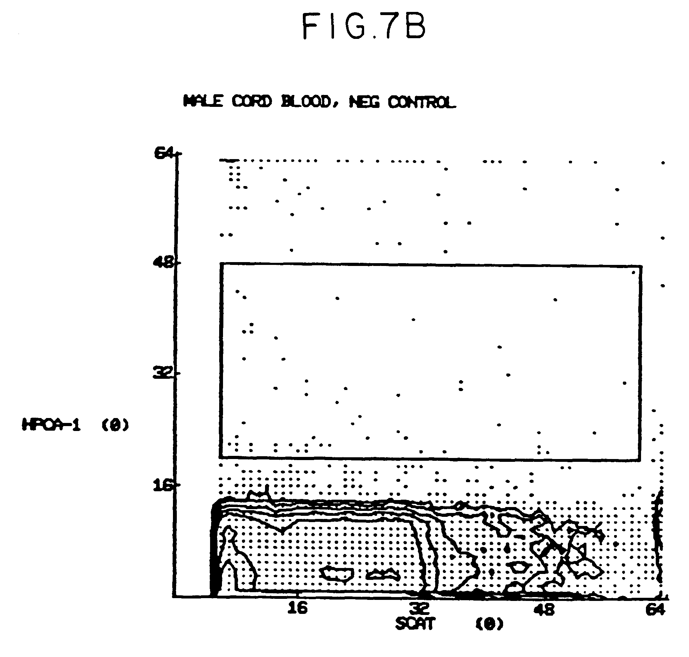

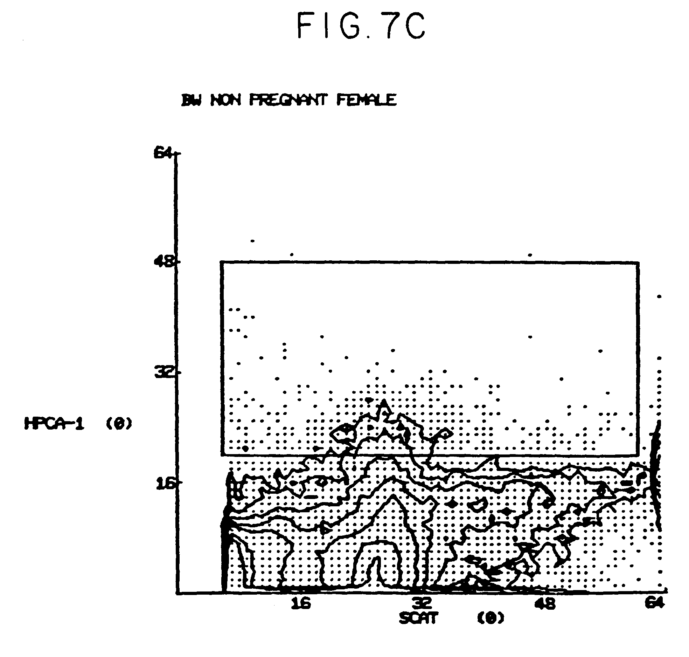

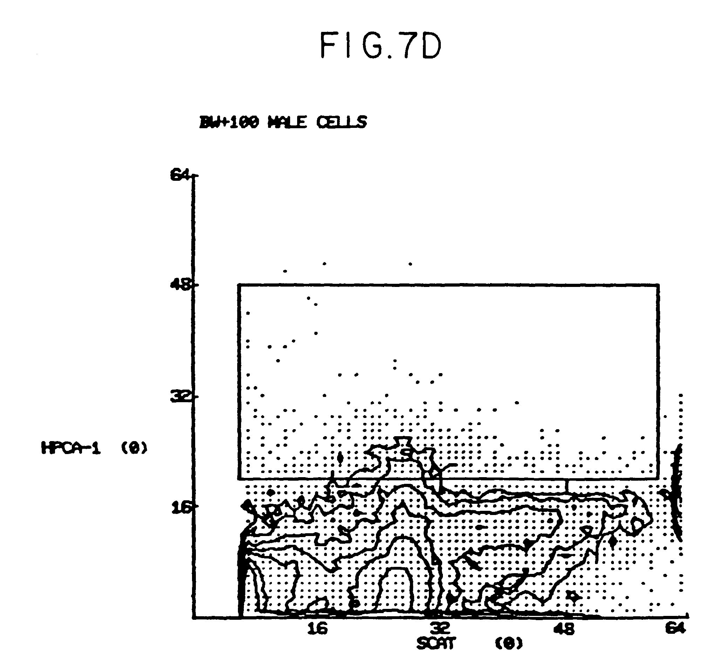

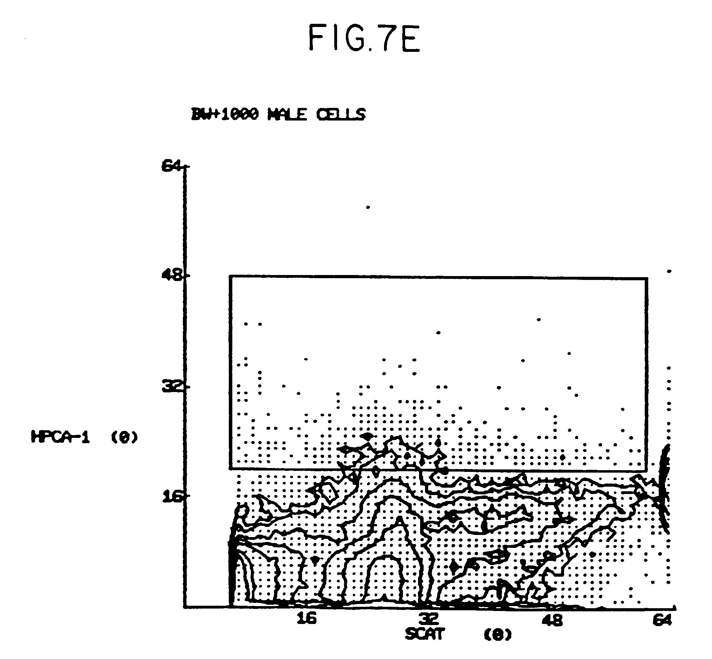

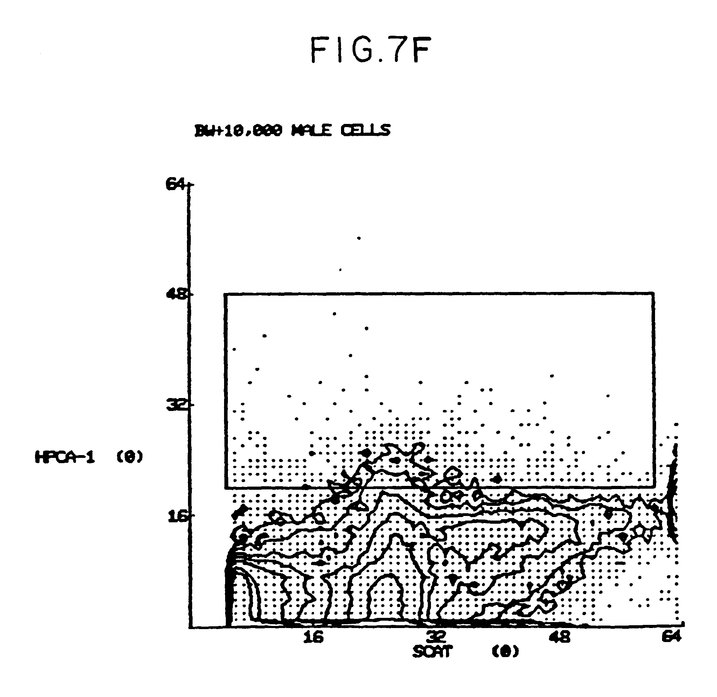

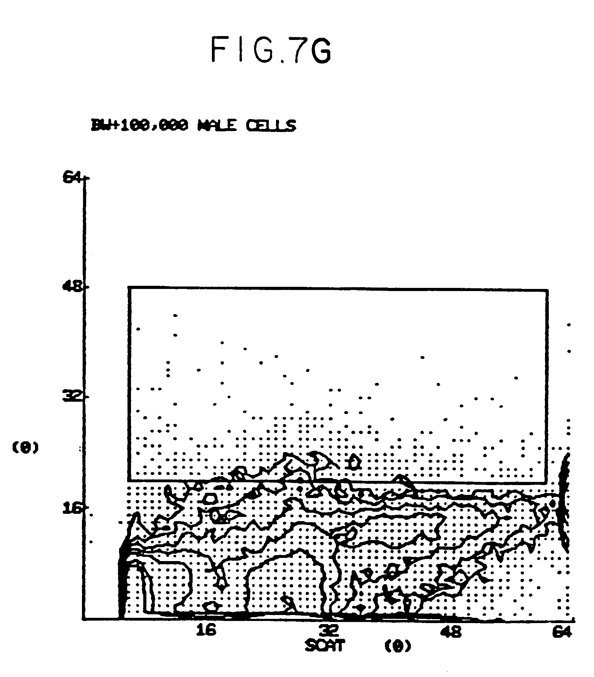

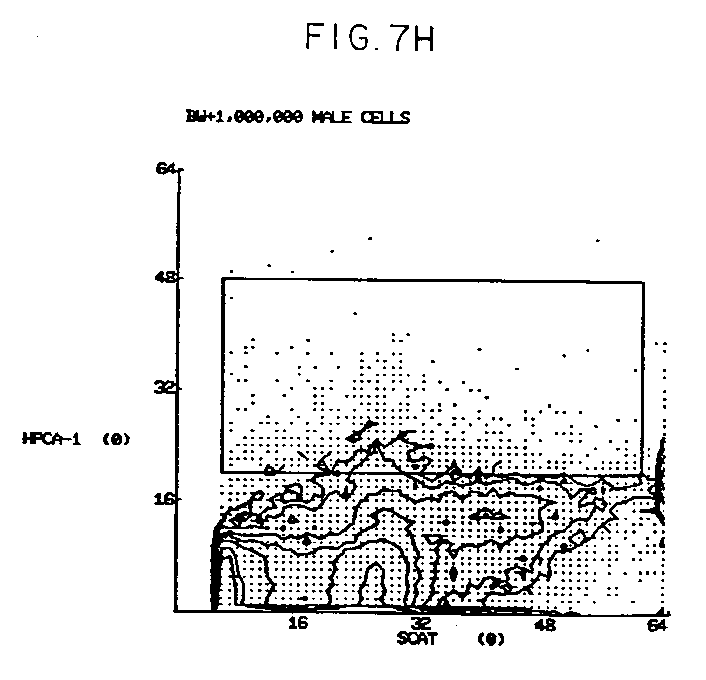

Figure 7 shows the histograms obtained when anti HPCA-1 antibody is used. In the non-pregnant female, 0.9% of the mononuclear cells react with antibody. In umbilical cord blood, a well-defined population of cells is seen, but the percentage is only 1.1%. Thus, the addition of umbilical cord blood cells to the non-pregnant female cells is not seen on the histograms as clearly as with the transferrin receptor antibody. An increased number of HPCA⁺ cells were collected as the amounts of added cord blood cells increased.

In agarose gels, the 397 bp band consistent with DNA was detected in the HPCA⁺ cells when 10³-10⁵ male cells were added to the female cells. Male DNA was detected in agarose gels in the HPCA cells when 10⁶ male cells were added to the female cells.

### Example 6 In situ Hybridization Using Molecular Probes Recognizing Individual Chromosomes in Flow Sorted Nucleated Erythrocytes

To demonstrate diagnostic utility of the present invention, a DNA probe set was constructed of chromosome specific probes that provided both good signal to noise ratios and good spatial resolution of the fluorescent signals. Accordingly, specific probes were developed for five chromosomes frequently seen as liveborn aneuploidies; chromosomes 13, 18, 21, X and Y. A probe for chromosome 1 was used as a control. In constructing the probes, the general strategy was to identify a starting clone that mapped to the desired chromosomal region by multiple genetic and physical methods, and then to use that clone to identify a matching cosmid "contig" which was then used as a hybridization probe.

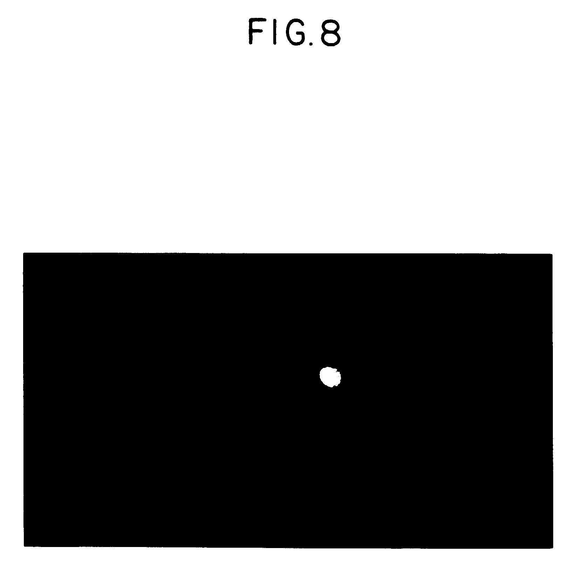

Hybridization of the high copy number repeat sequences was suppressed by inclusion of total genomic human DNA, and the chromosomal specificity verified by hybridization to metaphase spreads. The probes gave sharp, punctate fluorescent signals in interphase cells that was easily discriminated and enumerated. The Y probe used in this study was pDP97, a repetitive clone (a 5.3 kb EcoRI Y fragment from cosmid Y97 subcloned into EcoRI site of pUC-13). All probes were labeled with biotin, hybridized under suppression conditions, and specific hybridization detected by conjugated streptoavidin-FITC, which showed as a single "dot" in the FITC image. As illustrated in Figure 8, the Y chromosome was detected by in situ hybridization of the pDP97 probe for the Y chromosome in a fetal nucleated red blood cell. Thus, prenatal diagnosis for chromosomal abnormalities could be performed on fetal cells isolated from maternal blood.

## Claims

1. A noninvasive method for prenatal diagnosis of a chromosomal abnormality in a fetus, comprising the steps of:
(a) obtaining a maternal blood sample;
(b) treating the maternal blood sample to produce a blood sample enriched in fetal nucleated erythrocytes;
(c) performing in situ hybridization on the enriched sample with a chromosome specific probe or a probe specific for abnormal chromosomal DNA to generate spatially resolved signals in individual chromosomes of interphase cells; and
(d) using the signals generated in step (c) as the basis for diagnosis of a chromosomal abnormality in the fetus.

2. The method of claim 1 wherein the maternal blood sample is a maternal peripheral blood sample.

3. The method of claim 1 or claim 2 wherein the chromosomal abnormality is an aneuploidy.

4. The method of claim 1 or claim 2 wherein the in situ hybridization is fluorescent in situ hybridization.

5. The method of claim 1 or claim 2 wherein the probe is hybridizable to fetal DNA of interest contained in a chromosome selected from chromosomes 13, 18, 21, X and Y.

6. The method of any one of claims 1-5 wherein the treatment step (b) comprises a two step enrichment process.

7. The method of claim 6 wherein the two step enrichment includes the separation of non-nucleated cells from nucleated cells in the maternal sample forming a nucleated cell enriched sample and the treatment of the nucleated cell enriched sample such that the proportion of fetal cells in the sample is increased with respect to the proportion of maternal cells forming a sample enriched in fetal nucleated erythrocytes.

8. The method of claim 1 or claim 2 wherein the maternal sample is enriched in fetal nucleated cells using density gradient centrifugation.

9. The method of claim 1 or claim 2 wherein the sample is treated for enrichment for fetal nucleated erythrocytes using only a single fetal-specific monoclonal antibody.

10. The method of claim 1 or claim 2 wherein the sample is treated for enrichment for fetal nucleated erythrocytes using at least one fetal-specific monoclonal antibody.

11. The method of claim 1 or claim 2 wherein in step (c) the sample is supported on a solid support, for example a slide.

12. The method of claim 1 or claim 2 wherein the sample of maternal blood is treated by contacting it with a first monoclonal antibody which recognizes fetal nucleated erythrocytes but not cells derived from the maternal blood and/or a second monoclonal antibody which recognizes cells derived from the maternal blood but not fetal nucleated erythrocytes, under conditions appropriate for antibody binding thereby producing fetal nucleated erythrocyte-first monoclonal antibody complexes and/or maternal cell-second monoclonal antibody complexes, respectively, and separating fetal nucleated erythrocyte-first monoclonal antibody complexes from maternal cells and/or separating maternal cell-second monoclonal antibody complexes from fetal nucleated erythrocytes thereby separating fetal nucleated erythrocytes from cells derived from the maternal blood.

13. The method of claim 12 wherein the first and/or second monoclonal antibody is attached to a solid support.

14. The method of claim 1 or claim 2 wherein the maternal sample is obtained when the gestational age of the fetus is about fifteen to seventeen weeks.

15. The method of claim 14 wherein the maternal sample is obtained when the gestational age of the fetus is about 16 weeks.

16. The method of claim 13 wherein the solid support is an immunomagnetic bead.

17. The method of any one of claims 1, 4 or 6-16, wherein the probe is hybridizable to fetal DNA of interest contained in a Y chromosome.

18. The method of any one of claims 1, 4 or 6-16, wherein the probe is hybridizable to fetal DNA of interest which is associated with a disease.

19. The method of claim 18 wherein the disease is cystic fibrosis.

20. The method of claim 18 wherein the disease is Duchenne muscular dystrophy.

21. The method of claim 18 wherein the disease is haemophilia A.

22. The method of claim 18 wherein the disease is Gaucher's disease.

23. The method of claim 18 wherein the disaese is sickle cell anemia.

24. The method of claim 18 wherein the disease is β thalassemia.

25. The method of claim 18 wherein the disease is phenylketonuria.

## Patentansprüche

1. Nichtinvasives Verfahren zur pränatalen Diagnose einer Chromosomenanomalie in einem Fötus in den Schritten:
(a) Beschaffung einer Blutprobe der Mutter;
(b) Behandlung der Blutprobe der Mutter zur Herstellung einer mit kernhaltigen fötalen Erythrocyten angereicherten Blutprobe;
(c) Durchführung einer in situ-Hybridisierung an der angereicherten Probe mit einer chromosomenspezifischen Sonde oder einer für anomale chromosomale DNA spezifischen Sonde zur Erzeugung von Signalen mit räumlicher Auflösung in einzelnen Chromosomen von Interphasezellen; und
(d) Verwendung der in Schritt (c) erzeugten Signale als Grundlage für die Diagnose einer Chromosomenanomalie im Fötus.

2. Verfahren nach Anspruch 1, in welchem die Blutprobe der Mutter eine Blutprobe aus dem peripheren Körperbereich der Mutter ist.

3. Verfahren nach Anspruch 1 oder 2, in welchem die Chromosomenanomalie eine Aneuploidie ist.

4. Verfahren nach Anspruch 1 oder 2, in welchem die in situ-Hybridisierung eine in situ-Hybridisierung mit Fluoreszenzmarkierung ist.

5. Verfahren nach Anspruch 1 oder 2, in welchem die Sonde an betreffende fötale DNA hybridisierbar ist, welche in einem aus den Chromosomen 13, 18, 21, X und Y ausgewählten Chromosom enthalten ist.

6. Verfahren nach jedem der Ansprüche 1 bis 5, in welchem der Behandlungsschritt (b) eine Anreicherung in zwei Schritten umfaßt.

7. Verfahren nach Anspruch 6, in welchem die Anreicherung in zwei Schritten aus der Abtrennung kernfreier Zellen von kernhaltigen Zellen im Blut der Mutter unter Gewinnung einer mit kernhaltigen Zellen angereicherten Probe besteht, sowie aus der Behandlung der mit kernhaltigen Zellen angereicherten Probe derart, daß der Anteil an fötalen Zellen in der Probe in Bezug auf den Anteil der Zellen der Mutter, die eine an fötalen kernhaltigen Erythrocyten angereicherte Probe bilden, erhöht ist.

8. Verfahren nach Anspruch 1 oder 2, in welchem die Probe der Mutter unter Einsatz einer Dichtegradientenzentrifugation mit fötalen, kernhaltigen Zellen angereichert wird.

9. Verfahren nach Anspruch 1 oder 2, in welchem die Probe zur Anreicherung mit fötalen kernhaltigen Erythrocyten unter Verwendung nur eines einzigen fötusspezifischen monoklonalen Antikörpers behandelt wird.

10. Verfahren nach Anspruch 1 oder 2, in welchem die Probe zur Anreicherung mit fötalen kernhaltigen Erythrocyten unter Verwendung von mindestens einem fötusspezifischen monoklonalen Antikörpers behandelt wird.

11. Verfahren nach Anspruch 1 oder 2, in welchem in Schritt (c) die Probe auf einen festen Träger, beispielsweise einen Objektträger aufgetragen wird.

12. Verfahren nach Anspruch 1 oder 2, in welchem die Probe mit dem Blut der Mutter behandelt wird, indem man sie mit einem ersten monoklonalen Antikörper in Kontakt bringt, der fötale kernhaltige Erythrocyten, jedoch keine vom Blut der Mutter stammenden Zellen erkennt und/oder mit einem zweiten monoklonalen Antikörper, der von dem Blut der Mutter stammende Zellen erkennt, jedoch keine fötalen kernhaltigen Erythrocyten, wobei die Behandlung unter für die Antikörperbindung geeigneten Bedingungen erfolgt, wobei Komplexe des ersten monoklonalen Antikörpers mit fötalen kernhaltigen Erythrocyten und/oder entsprechend Komplexe des zweiten monoklonalen Antikörpers mit Zellen der Mutter gebildet werden und wobei die Komplexe des ersten monoklonalen Antikörpers mit den fötalen kernhaltigen Erythrocyten von Zellen der Mutter abgetrennt und/oder Komplexe des zweiten monoklonalen Antikörpers mit Zellen der Mutter von fötalen kernhaltigen Erythrocyten abgetrennt werden, wodurch eine Trennung von fötalen kernhaltigen Erythrocyten von vom Blut der Mutter stammenden Zellen erfolgt.

13. Verfahren nach Anspruch 12, in welchem der erste und/oder zweite monoklonale Antikörper an einen festen Träger angeheftet wird.

14. Verfahren nach Anspruch 1 oder 2, in welchem die Probe der Mutter gewonnen wird, wenn das Schwangerschaftsalter des Fötus ca. 15 bis 17 Wochen beträgt.

15. Verfahren nach Anspruch 14, in welchem die Probe der Mutter gewonnen wird, wenn das Schwangerschaftsalter des Fötus ca. 16 Wochen beträgt.

16. Verfahren nach Anspruch 13, in welchem der feste Träger ein immunomagnetisches Kügelchen ist.

17. Verfahren nach einem der Ansprüche 1, 4 oder 6 bis 16, in welchem die Sonde an in einem Y-Chromosom enthaltene betreffende fötale DNA hybridisierbar ist.

18. Verfahren nach einem der Ansprüche 1, 4 oder 6 bis 16, in welchem die Sonde an betreffende fötale DNA hybridisierbar ist, die in Verbindung mit einer Krankheit steht.

19. Verfahren nach Anspruch 18, in welchem die Krankheit Mukoviszidose ist.

20. Verfahren nach Anspruch 18, in welchem die Krankheit eine Muskeldystrophie des Duchenne-Typs ist.

21. Verfahren nach Anspruch 18, in welchem die Krankheit eine Hämophilie A ist.

22. Verfahren nach Anspruch 18, in welchem die Krankheit die Gaucher-Krankheit ist.

23. Verfahren nach Anspruch 18, in welchem die Krankheit eine Sichelzellenanämie ist.

24. Verfahren nach Anspruch 18, in welchem die Krankheit eine β-Thalassämie ist.

25. Verfahren nach Anspruch 18, in welchem die Krankheit eine Phenylketonurie ist.

## Revendications

1. Méthode non invasive de diagnostic prénatal d'une anomalie chromosomique dans un foetus, comprenant les étapes consistant à :
(a) se procurer un échantillon de sang maternel ;
(b) traiter l'échantillon de sang maternel de manière à produire un échantillon de sang enrichi en érythrocytes nucléés foetaux ;
(c) réaliser une hybridation in situ sur l'échantillon enrichi avec une sonde spécifique du chromosome ou une sonde spécifique d'ADN chromosomique anormal de manière à produire des signaux à résolution spatiale dans des chromosomes individuels de cellules en interphase ; et
(d) utiliser les signaux produits à l'étape (c) en vue d'un diagnostic d'une anomalité chromosomique dans le foetus.

2. Méthode selon la revendication 1, dans laquelle l'échantillon de sang maternel est un échantillon de sang périphérique maternel.

3. Méthode selon la revendication 1 ou la revendication 2, dans laquelle l'anomalie chromosomique est une hétéroploïdie.

4. Méthode selon la revendication 1 ou la revendication 2, dans laquelle l'hybridation in situ est une hybridation in situ fluorescente.

5. Méthode selon la revendication 1 ou la revendication 2, dans laquelle la sonde est capable de s'hybrider à l'ADN foetal d'intérêt contenu dans un chromosome choisi dans le groupe constitué par les chromosomes 13, 18, 21, X et Y.

6. Méthode selon l'une quelconque des revendications 1 à 5 dans laquelle l'étape de traitement (b) comprend un procédé d'enrichissement à deux étapes.

7. Méthode selon la revendication 6 dans laquelle l'enrichissement à deux étapes comprend la séparation de cellules anucléées des cellules nucléées dans l'échantillon maternel formant un échantillon enrichi en cellules nucléées et le traitement de l'échantillon enrichi en cellules nucléées de sorte que la proportion de cellules foetales dans l'échantillon soit augmentée par rapport à la proportion de cellules maternelles formant un échantillon enrichi en érythrocytes nucléés foetaux.

8. Méthode selon la revendication 1 ou la revendication 2 dans laquelle l'échantillon maternel est enrichi en cellules nucléées foetales en utilisant une centrifugation en gradient de densité.

9. Méthode selon la revendication 1 ou la revendication 2 dans laquelle l'échantillon est traité en vue de l'enrichissement en érythrocytes nucléés foetaux en utilisant un anticorps monoclonal unique spécifique du foetus.

10. Méthode selon la revendication 1 ou la revendication 2 dans laquelle l'échantillon est traité en vue de l'enrichissement en érythrocytes nucléés foetaux en utilisant au moins un anticorps monoclonal spécifique du foetus.

11. Méthode selon la revendication 1 ou la revendication 2 dans laquelle à l'étape (c) l'échantillon est maintenu sur un support solide tel qu'une lamelle, par exemple.

12. Méthode selon la revendication 1 ou la revendication 2 dans laquelle l'échantillon de sang maternel est traité par un procédé comprenant les étapes consistant à mettre en contact l'échantillon, avec un premier anticorps monoclonal qui reconnaît les érythrocytes nucléés foetaux mais pas les cellules provenant du sang maternel et / ou avec un deuxième anticorps monoclonal qui reconnaît des cellules provenant du sang maternel mais pas des érythrocytes nucléés foetaux, dans des conditions qui conviennent à la fixation de l'anticorps produisant ainsi des complexes érythrocytes nucléés foetaux - premier anticorps monoclonal et / ou des complexes cellule maternelle - deuxième anticorps monoclonal, respectivement, et à séparer les complexes érythrocytes nucléés foetaux - premier anticorps monoclonal des cellules maternelles et / ou à séparer les complexes cellule maternelle - deuxième anticorps monoclonal des érythrocytes nucléés foetaux séparant ainsi les érythrocytes nucléés foetaux des cellules provenant du sang maternel.

13. Méthode selon la revendication 12 dans laquelle le premier et / ou le deuxième anticorps monoclonal est fixé sur un support solide.

14. Méthode selon la revendication 1 ou la revendication 2 dans laquelle l'échantillon maternel est obtenu lorsque l'âge gestationnel du foetus est environ quinze à dix-sept semaines.

15. Méthode selon la revendication 14, dans laquelle l'échantillon maternel est obtenu lorsque l'âge gestationnel du foetus est environ 16 semaines.

16. Méthode selon la revendication 13 dans laquelle le support solide est une bille immunomagnétique.

17. Méthode selon l'une quelconque des revendications 1, 4 ou 6 à 16, dans laquelle la sonde peut s'hybrider à l'ADN foetal d'intérêt contenu dans un chromosome Y.

18. Méthode selon l'une quelconque des revendications 1, 4 ou 6 à 16, dans laquelle la sonde peut s'hybrider à l'ADN foetal d'intérêt qui est associé à une maladie.

19. Méthode selon la revendication 18 dans laquelle la maladie est la fibrose kystique.

20. Méthode selon la revendication 18 dans laquelle la maladie est la dystrophie musculaire de Duchenne.

21. Méthode selon la revendication 18 dans laquelle la maladie est l'hémophilie A.

22. Méthode selon la revendication 18 dans laquelle la maladie est la maladie de Gaucher.

23. Méthode selon la revendication 18 dans laquelle la maladie est la drépanocytose.

24. Méthode selon la revendication 18 dans laquelle la maladie est la thalassémie β.

25. Méthode selon la revendication 18 dans laquelle la maladie est la phénylcétonurie.
